# EUROPEAN PATENT APPLICATION

(11) **EP 4 101 373 A1**
(43) Date of publication of application: **14.12.2022**
(21) Application number: 22382542.3
(22) Date of filing: 06.06.2022
(51) Int. Cl.: A61B 5/00

(54) **SYSTEM AND COMPUTER-IMPLEMENTED METHOD FOR THE QUANTITATIVE ASSESSMENT OF PAIN**

(30) Priority: 07.06.2021 ES 202131175 U
(71) Applicant: UPintelligence S.L., 33011 Oviedo (ES)
(72) Inventor: COSIDO COBOS, Oscar Jesús, 33211 Gijón (ES)
(74) Representative: Clarke Modet & Co.

(57) **Abstract**

System and computer-implemented method for the quantitative assessment of pain. The system comprises an element (1) for exerting pressure on a part of the body of a patient (11) with a pressure sensor (2), an interface (4) for displaying in a graphic representation (6) the part of the body where the element (1) is applied, a processing unit (3) for receiving the data about the measurement of the pressure sensor (2), and associating said data with the part of the body to which it corresponds and with a pain value on a pain scale, wherein said pain value is provided by the patient (11), and for sending information about the pain value and the pressure measured to the interface (4) so as to be displayed in the graphic representation (6).

## Description

### Technical field of the invention

The invention belongs to the field for characterizing the pain experienced by a patient. More specifically, it relates to techniques for assessing pain using a graphic representation of the patient's perception of pain by means of the controlled application of a stimulus.

### State of the art

Systems today are for the most part qualitative: the healthcare professional presses on part of the individual's body and the individual responds by indicating his or her sensation of pain. Pain pens intend to quantify and increase the objectivity of the measurements through systems which allow the pressure applied by the healthcare professional to be quantified at all times.

### Brief description of the invention

It would be desirable to generate pain maps based on the area of the body being assessed, the pressure exerted and the discomfort expressed by the patient.

An object of the present invention is a system generally defined by independent claim 1. An object of the present invention is a computer-implemented method generally defined by the independent claim 9. Several particular embodiments are also described in the dependent claims.

A system is proposed for the generation of pain maps based on the controlled application of pressure on the patient's skin and software which allows the datum to be sensed and transformed into a pain map in an instant of time.

The system is made up of a pressure sensor, a user interface, a processing unit with data storage capability. The system allows, between other tasks, the selection of the contact area of the body to be performed, the measurement of pressure by the sensor to be received, and the values relating to the patient's perception to be recorded. Namely, the processing unit receives the data about the measurement of the pressure sensor. It associates and records said data with the part of the body of the patient to which it corresponds and with a pain value on a pain scale. Said pain value is provided by the patient. The processing unit sends information about the pain value and the pressure measured to the interface so as to be displayed in the graphic representation of the interface. The processing unit sends data about the measurement to the interface so as to be displayed in the graphic representation.

The system stores the pressure signal generated by an element for exerting pressure, such as a pen, the area of the human body where the measurement is taken (entered by the healthcare personnel according to a map of the body), and the response provided by the patient about his or her sensation of pain (for example, how it is perceived on a scale of 1 to 10).

Namely, the element for exerting pressure has a threshold setting to start measuring with the pressure sensor.

The software transforms the data into a graphic representation referred to as a pain map of an individual, which map will be generated again in subsequent sessions for the quantitative assessment of pain of the same patient for having time series which allow a better measurement of the progression of the pain of said patient to be performed, by lower subjectivity.

Additionally, the system can incorporate data from other medical devices such as pulse oximeters or pressure meters. Said physiological data (blood pressure, pulse, blood oxygen, etc.) are combined with the user's response in each measurement for the purpose of increasing objectivity of the method.

The system can have a warning unit for outputting a warning which establishes the start and end of the interval for applying the element for exerting pressure. The warning can be audible, luminous, vibratory or a combination of any of the foregoing.

### Brief description of the figures

To complement the description provided herein, and for the purpose of helping to make the features of the invention more readily understandable, said description is accompanied by a set of drawings constituting an integral part of the same, which by way of illustration and not limitation represents the following:
FIG. 1 shows a functional block diagram according to the invention.
FIG. 2 shows an embodiment of the pressure element.
FIG. 3 shows an example representation on the interface according to the invention.

### Detailed description of the invention

An exemplary embodiment of the system is described in greater detail in reference to the above figures.

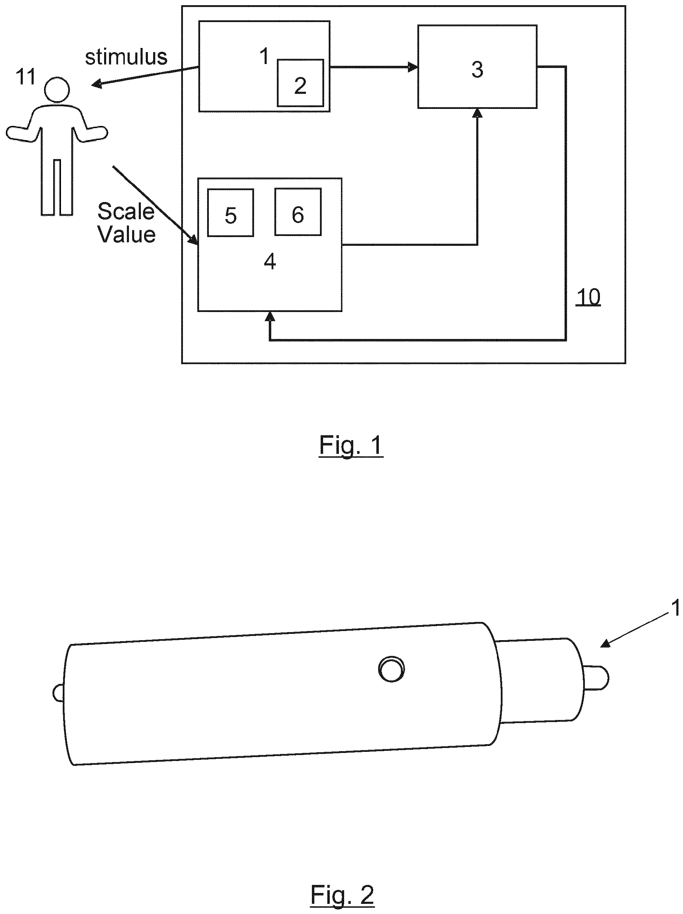

In FIG. 1, a simplified diagram of the invention can be seen. The system 10 allows the intensity of pain in a patient 11 to be recorded and analysed. To that end, it uses as a stimulus the pressure exerted by means of a sharp pointed element 1 referred to as a pen. This element 1 has an associated pressure sensor 2 and is preferably handled by a healthcare professional. The response of the patient 11 is established in reference to a scale with several levels, for example, between 1 and 10. The system 10 has an interface 4 for selecting, by means of a graphic representation 6, the area of the body being worked on. The system 10 records, together with the level reached on the scale indicated by the patient 11, the pressure exerted measured by the sensor 2 and the area where pressure was applied.

The system thus obtains a pain map showing three variables: the area of contact on the body, the pressure exerted and the pain scale expressed by the patient. The manner in which the data is communicated is preferably wirelessly (e.g. Bluetooth).

The system can incorporate a warning unit for outputting a luminous, audible or vibratory signal which appropriately marks the start and end of the interval during which the element 1 for exerting pressure is to be applied. The usability and reproducibility of the session is thereby facilitated.

FIG. 2 shows an embodiment of the pressure element 1 as a pen which integrates a pressure sensor with wireless communication by Bluetooth.

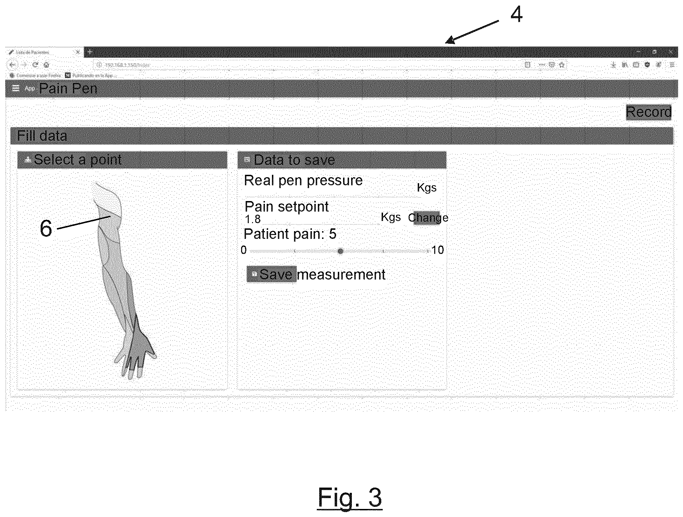

FIG. 3 shows an example interface 4 which can be implemented in the invention.

For example, the system can be implemented with the following components. A load cell with a range of 0 to 4.5 kg as the pressure sensor associated with a microcontroller for reading and conditioning the signal from the sensor, and a conventional computer or tablet for user interface functions and with data storage capability.

Optionally, the system can correlate the perception of pain expressed by the patient with several physiological parameters directly related to real pain. The intention is to study the real intensity of pain in patients by means of analysing their internal conditions, such as blood pressure, heart rate, skin conductivity, blood oxygen content, etc These parameters are measured by means of other portable (wearable) measurement devices. For example, with additional medical instruments such as a pulse meter, a pulse oximeter, a sphygmomanometer, galvanic skin resistance sensor, etc.

The processing unit 3 compiles and records this data. Time series of said data are correlated with the subjective level of pain provided by the patient with an installed software application using data mining techniques. A uniform criterion can be obtained and the magnitude of pain can be estimated objectively by using artificial intelligence techniques. Treatments and analgesics can thus be adapted for each patient.

Advantageously, the system allows a standardised, objective, and completely computerised collection of data from patients. A thorough definition of groups of patients with characteristics suitable for decision making in terms of a patient referral to pain treatment units.

Benefits for the patient's quality of life in terms of providing specialised treatments adapted to the particular characteristics and needs, involving positive consequences for the person and for his or her immediate surroundings.

The industrial application of the invention is clear and extensive. Various entities such as public and private clinics (physical therapy, rehabilitation), medical insurance companies, mutual health insurance companies, can utilise the advantages of an objective assessment of pain for use in treatments, compensations and expert evidence.

The invention can similarly be implemented as a computer-implemented method for the quantitative assessment of pain. This method includes various steps which can be run by a computer or the like. The main steps are the following:
A step for exerting pressure on a part of the body of a patient 11 by means of element 1.

A step for measuring the pressure exerted by means of a pressure sensor 2 comprised in said element 1.

A step for displaying in a graphic representation 6, by means of an interface 4, the part of the body of the patient 11 where the element 1 is applied.

A step for receiving the data about the measurement from the pressure sensor 2 by means of a processing unit 3.

A step for recording and associating said data, by means of the processing unit 3, with the part of the body of the patient 11 and with a pain value on a pain scale, and said pain value is provided by the patient 11.

A step for sending information about the pain value and about the pressure measured to the interface 4.

A step for displaying the preceding information in the graphic representation 6.

This method, in turn, can be coded in program instructions and saved in computer-readable storage means, such as a DVD, CD, a solid-state memory, etc.

### NUMERICAL REFERENCES

- 1: Element for applying pressure (for example, a pen).
- 2: Pressure sensor.
- 3: Processing unit.
- 4: Interface.
- 5: Warning unit.
- 6: Graphic representation.
- 10: System for the quantitative assessment of pain.
- 11: Patient.

## Claims

1. A system for the quantitative assessment of pain, comprising:
an element (1) for exerting pressure on a part of the body of a patient (11), where said element comprises a pressure sensor (2) for measuring the pressure exerted;
an interface (4) for displaying in a graphic representation (6) the part of the body of the patient (11) where the element (1) is applied;
a processing unit (3) for receiving the data about the measurement of the pressure sensor (2), and for recording and associating said data with the part of the body of the patient (11) to which it corresponds and with a pain value on a pain scale, wherein said pain value is provided by the patient (11), and for sending information about the pain value and the pressure measured to the interface (4) so as to be displayed in the graphic representation (6) of the interface (4).

2. The system for the quantitative assessment of pain according to claim 1, wherein the element (1) for exerting pressure has a threshold setting to start measuring with the pressure sensor (2).

3. The system for the quantitative assessment of pain according to claim 1 or 2, wherein the interface (4) comprises a warning unit (5) for outputting a warning which establishes the start and end of the interval for applying the element (1) for exerting pressure.

4. The system for the quantitative assessment of pain according to claim 3, wherein the warning is audible, luminous, vibratory or a combination of any of the above.

5. The system for the quantitative assessment of pain according to any one of the preceding claims, wherein the element for exerting pressure comprises a wireless communication unit to send the pressure measured by the pressure sensor (2).

6. The system for the quantitative assessment of pain according to any one of the preceding claims, further comprising at least one of the following devices:
a pulse oximeter for measuring the heart rate and blood oxygen saturation of the patient (11);
a pulse meter for measuring the heart rate of the patient (11);
a sphygmomanometer for measuring the blood pressure of the patient (11);
a galvanic skin resistance sensor for measuring the skin conductivity of the patient (11);
wherein the processing unit (3) receives data about the measurement, associates and records said data with the pain value on the pain scale provided by the patient.

7. The system for the quantitative assessment of pain according to claim 6, wherein the processing unit (3) sends data about the measurement to the interface (4) to be displayed in the graphic representation (6).

8. The system for the quantitative assessment of pain according to claim 6 or 7, wherein the processing unit (3) correlates time series of data measured together with the pain values provided by the patient to estimate the magnitude of pain.

9. A computer-implemented method for the quantitative assessment of pain comprising steps for:
exerting pressure on a part of the body of a patient (11) by means of an element (1);
measuring the pressure exerted by means of a pressure sensor (2) comprised in said element (1);
displaying in a graphic representation (6), by means of an interface (4), the part of the body of the patient (11) where the element (1) is applied;
receiving the data about the measurement from the pressure sensor (2) by means of a processing unit (3);
recording and associating said data, by means of the processing unit (3), with the part of the body of the patient (11) to which it corresponds and with a pain value on a pain scale, wherein said pain value is provided by the patient (11);
sending information about the pain value and about the pressure measured to the interface (4);
displaying said information in the graphic representation (6).

10. The method according to claim 9, wherein the step for exerting pressure by means of the element (1) includes setting the threshold to start measuring with the pressure sensor (2).

11. The method according to claim 9 or 10, further comprising a step for outputting a warning which establishes the start and end of the interval for applying the element (1) for exerting pressure by means of a warning unit (5) comprised in the interface (4).

12. The method according to any one of the preceding claims 9 to 11, wherein the step for exerting pressure comprises a step for sending the pressure measured by the pressure sensor (2) by means of a wireless communication unit.

13. The method according to any one of the preceding claims 9 to 12, further comprising at least one of the following steps:
measuring the heart rate and blood oxygen saturation of the patient (11) by means of a pulse oximeter;
measuring the heart rate of the patient (11) by means of a pulse meter;
measuring the blood pressure of the patient (11) by means of a sphygmomanometer;
measuring the skin conductivity of the patient (11) by means of a galvanic skin resistance sensor.

14. The method according to claim 13, further comprising a step for correlating time series of data measured together with the pain values provided by the patient to estimate the magnitude of pain by means of the processing unit (3).

15. A storage medium comprising program instructions which, when run by a computer, carry out the method for the quantitative assessment of pain according to any one of claims 9 to 14.
